# EUROPÄISCHE PATENTSCHRIFT

(11) **EP 0 761 585 B1**
(45) Veröffentlichungstag und Bekanntmachung des Hinweises auf die Patenterteilung: **02.02.2000**
(21) Anmeldenummer: 96113517.5
(22) Anmeldetag: 23.08.1996
(51) Int. Cl.: B65H 63/06

(54) **Garnsensor**
Yarn sensor
Détecteur de fil

(30) Priorität: 06.09.1995 CH 252395
(43) Veröffentlichungstag der Anmeldung: 12.03.1997
(73) Patentinhaber: ZELLWEGER LUWA AG, 8610 Uster (CH)
(72) Erfinder: Schilling, Peter, 8854 Siebnen (CH); Bucher, Cyrill, 8304 Wallisellen (CH)
(74) Vertreter: Ellenberger, Maurice

(56) Entgegenhaltungen:
- EP-A- 0 425 015
- EP-A- 0 553 445
- WO-A-93/13407
- WO-A-93/19359

## Beschreibung

Die Erfindung betrifft einen Gamsensor zum Abtasten eines, in seiner Längsrichtung in einem Messspalt, bewegten Garns mit einem Lichtstrahl aus einer Lichtquelle, mit einem ersten Empfänger für direkt übertragenes Licht, Elementen zum Übertragen des Lichts zwischen dem Messspalt, der Lichtquelle und dem Empfänger, wobei die optischen Achsen der Elemente zum Übertragen des Lichts zusammmen in einer Ebene liegen, die quer zum Garn steht.

Aus der EP-A-0 425 015 ist eine Vorrichtung für das kontinuierliche Erfassen von Abmessungen eines länglichen Prüfgutes bekannt, bei der zwei Lichtquellen etwa in einem rechten Winkel in einer Ebene senkrecht zur Hauptachse des Prüfguts in Längsrichtung angeordnet sind. Jeder Lichtquelle steht jenseits des Prüfguts ein Empfänger gegenüber. Damit kann beispielsweise der Durchmesser des Prüfguts in zwei Richtungen erfasst werden.

Ein Nachteil dieser Vorrichtung ist beispielsweise darin zu sehen, dass sie nur für die Erfassung des Durchmessers des Prüfguts geeignet ist. Andere Messungen, die z.B. angeben könnten ob Fremdstoffe oder Fremdfasem im Prüfgut vorhanden sind, können damit nicht gemacht werden.

Aus der WO-A-93/13407 ist ein Gamsensor bekannt, bei dem ein Empfänger für übertragenes Licht und ein Empfänger für reflektiertes Licht einander gegenüberliegend beidseits eines Messspaltes angeordnet sind. Auf einer Seite des Messspaltes sind zudem Eintritts- und Austrittsprismen für das Licht, in Richtung des Garns gesehen, nebeneinander aufgereiht.

Ein Nachteil dieses bekannten Gamsensors besteht darin, dass die verschiedenen Elemente, die zum Übertragen und ortsgerechten Einleiten von Licht vorgesehen sind, viel Platz beanspruchen. Solche Gamsensoren sind aber zum Einbau in Textilmaschinen vorgesehen wo oft nur wenig Platz dafür frei ist. Es kann demnach vorkommen, dass solche bekannten Gamsensoren in bestimmten Textilmaschinen gar nicht eingebaut werden können.

Gegenstand der Erfindung ist deshalb ein Gamsensor, der nach aussen nur wenig Platz beansprucht, für verschiendenartige Messungen geeignet ist und auch mit einem relativ kleinen Messspalt ausgerüstet sein kann.

Erfindungsgemäss wird dies dadurch erreicht, dass die optischen Achsen der Elemente zum Übertragen des Lichts zwischen dem Garn und den Empfängern zusammmen in einer Ebene liegen, die quer oder vorzugsweise mindestens annähernd senkrecht zum Garn steht, oder die vom Garn durchstossen wird, wobei neben einem Empfänger für Durchlicht, der die Abschattung der Lichtquelle durch das Garn misst, mindestens zwei weitere Empfänger für Licht vorgesehen sind, das vom Garn reflektiert wird. Die optischen Achsen der weiteren Empfänger stehen ebenfalls quer zum Garn und schneiden sich im Bereiche des Garns. Diese Anordnung ergibt im Messspalt beispielsweise auf einer Seite ein Fenster für Licht zur Belichtung des Gams und, danebenliegend, Fenster für die Aufnahme von reflektiertem Licht, die eigentlich neben dem Garn liegen und so nur seitlich vom Garn reflektiertes Licht aufnehmen können.

Die durch die Erfindung erreichten Vorteile sind insbesondere darin zu sehen, dass das Messprinzip, wonach beispielsweise Fremdkörper oder Fremdfasern in einem Garn durch Auswertung des übertragenen Lichts und des reflektierten Lichts erkannt werden können, auch bei kleinen Messspalten angewendet werden kann. Dabei kann der Raumbedarf in der Umgebung des Messspaltes ebenfalls reduziert werden. Zudem kann der Messspalt in eine Schmalseite des Messkopfes oder Gamsensors eingebaut werden, was bedeutet, dass das Garn im Gamsensor nur einen kurzen Weg zurücklegt. Ein weiterer Vorteil ist darin zu sehen, dass gemäss der Erfindung der Messspalt klein sein kann und damit der bekannte Selbstreinigungseffekt des Messspaltes durch das Garn gut ausgenützt werden kann. Bei kleinen Messspalten vermindert sich auch der Einfluss von Fremdlicht, das durch die Eintrittsöffnung für das Gam eintreten kann. Bei kleinen Messspalten kommt die Messzone auch nahe an das Gam zu liegen, was die Messgenauigkeit steigert und die Messzone sauber hält.

Im folgenden wird die Erfindung anhand eines Beispiels und mit Bezug auf die beiliegenden Figuren näher erläutert. Es zeigen:
Figur 1 einen Schnitt durch einen Teil eines Gamsensors, in vereinfachter Darstellung,
Figur 2 eine schematische Darstellung eines Teils eines Messspaltes,
Figur 3 und 4 je ein Einzelteil des Garnsensors,
Figur 5 einen Signalverlauf einer Messfeldcharakteristik,
Figur 6 einen Schnitt durch einen Teil einer weiteren Ausführung eines Gamsensors und
Figur 7 eine schematische Darstellung möglicher Anordnungen von optischen Achsen in einem Garnsensor.

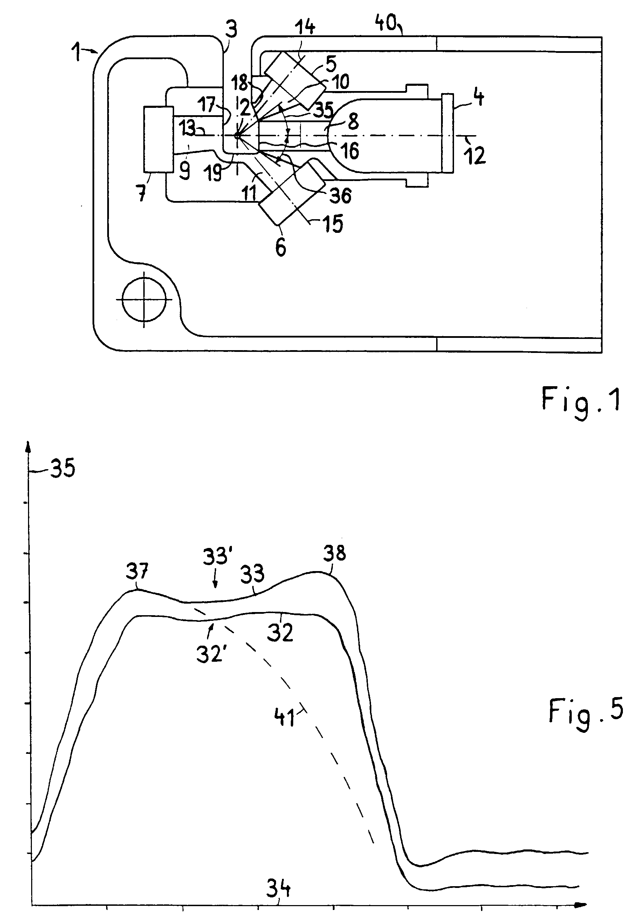

Fig. 1 zeigt in etwas vereinfachter Darstellung im Schnitt einen Garnsensor 1 für ein Garn 2 mit einem Messspalt 3. In dessen Umgebung sind eine Lichtquelle 4 und Empfänger 5 und 6 für reflektiertes Licht sowie ein Empfänger 7 für übertragenes Licht oder Durchlicht angeordnet. Die Lichtquelle 4 und die Empfänger 5, 6 und 7 sind an sich bekannte Bauelemente und deshalb hier nicht näher dargestellt. Elemente 8, 9, 10 und 11 zum Übertragen des Lichts sind zwischen dem Messspalt 3, der Lichtquelle 4 und den Empfängern 5, 6, 7 angeordnet. Diese Elemente 8 - 11 bestehen beispielsweise aus einem Lichtschacht mit viereckigem Querschnitt, der leer oder hohl sein kann oder der mit einem lichtleitenden oder lichtdurchlässigen Körper ausgefüllt sein kann. Die Wände, die den Lichtschacht begrenzen, sind verspiegelt oder Licht absorbierend ausgeführt. Jedes dieser Elemente 8 - 11 hat eine optische Achse 12, 13, 14 und 15, die sich vorzugsweise im Bereiche des Garns 2 schneiden, und von denen mindestens zwei zusammen eine Ebene, hier die Zeichnungsebene, aufspannen, die etwa senkrecht zur Achse des Garns 2 steht. Dort wo die Elemente 8 - 11 oder deren Lichtschächte in den Messspalt 3 münden, ergeben sich Fenster16, 17, 18 und 19, durch die Licht in den Messspalt 3 ein- oder austritt. In die Elemente 8 - 11 können auch Filter eingebaut sein, die das Spektrum des Lichts begrenzen. Beispielsweise können die Fenster 16 bis 19 als solche Filter ausgebildet sein.

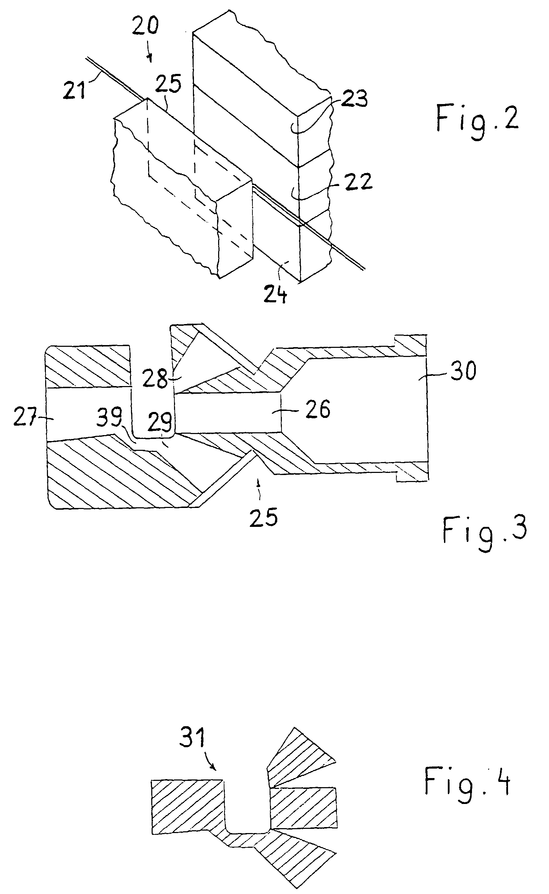

Fig. 2 zeigt schematisch einen Teil eines Messspaltes 20 für ein Garn 21 mit einem Fenster 22 zum Belichten des Garns 21, Fenstern 23 und 24 zum Empfangen des vom Garn 21 reflektierten Lichts und einem Fenster 25 zum Empfangen von Restlicht aus dem Fenster 22. Dieses Restlicht, ist dasjenige Licht, das aus dem Fenster 22 tritt und vom Garn 21 nicht abgedeckt, nicht zurückgehalten oder zurückgeworfen wird. Somit empfängt das Fenster 25 das Licht aus dem Fenster 22 vermindert um den Schatten den das Garn 21 auf das Fenster 25 wirft. Während die Fenster 22 und 25 vom Garn 21 abgedeckt werden oder dem Garn 21 gegenüberliegen, liegen die Fenster 23 und 24 ganz neben dem Garn 21 oder neben der optischen Achse 12 der Lichtquelle 4 (Fig. 1), wenn man das Garn etwa senkrecht zu seiner Bewegungsrichtung betrachtet. Fig. 2 zeigt die ursprüngliche oder artreine Anordung der Fenster 22 - 25 zueinander. In der Praxis kann es vorteilhaft sein, wie in Fig. 1 gezeigt, das Fenster 19 am Grund des Messspaltes 3 anzuordnen. Damit kann vermieden werden, dass der Messspalt 3 sich zuweit nach unten erstreckt, so dass der bekannte Effekt der Selbstreinigung des Messspaltes durch das Garn 2 ausgenützt werden kann.

Fig. 3 zeigt einen Teil 25 des Garnsensors 1, der einen Lichtschacht 26 für zugeführtes Licht und Lichtschächte 27, 28 und 29 für Licht, das vom Garn weggeführt ist bildet. Der Teil 25 weist auch eine Aufnahme 30 für eine Lichtquelle auf. Der Teil 25 ist einstückig ausgebildet, so dass damit alle aus der Fig. 1 bekannten Elemente 8 - 11 zum Übertragen des Lichts darin untergebracht werden können. Um dies zu ermöglichen ist hier auch eine kleine Brücke 39 zwischen den Lichtschächten 27 und 29 vorgesehen. An den äusseren Enden der Lichtschächte 27, 28 und 29 sind übliche, hier nicht gezeigte Empfänger vorgesehen.

Fig. 4 zeigt wiederum einem lichtleitenden oder lichtdurchlässigen Körper 31, der in den Teil 25 aus Fig. 3 eingesetzt werden kann und dort insbesondere als Einsatz in die Lichtschächte 26 bis 29 gedacht ist. Damit kann ein erfindungsgemässer Garnsensor aus den in Fig. 3 und 4 gezeigten beiden Teilen 25, 31 mit den zugeordneten Empfängern und der Lichtquelle aufgebaut sein. Diese Anordnung ist dann, wie aus Fig. 1 ersichtlich noch in einem Gehäuse unterzubringen. Die Elemente zum Übertragen des Lichts sind darin zusammen als einziger zusammenhängender Körper 25, 31 ausgebildet. Durch diese Anordnung wird die Montage des erfindungsgemässen Garnsensors stark erleichtert.

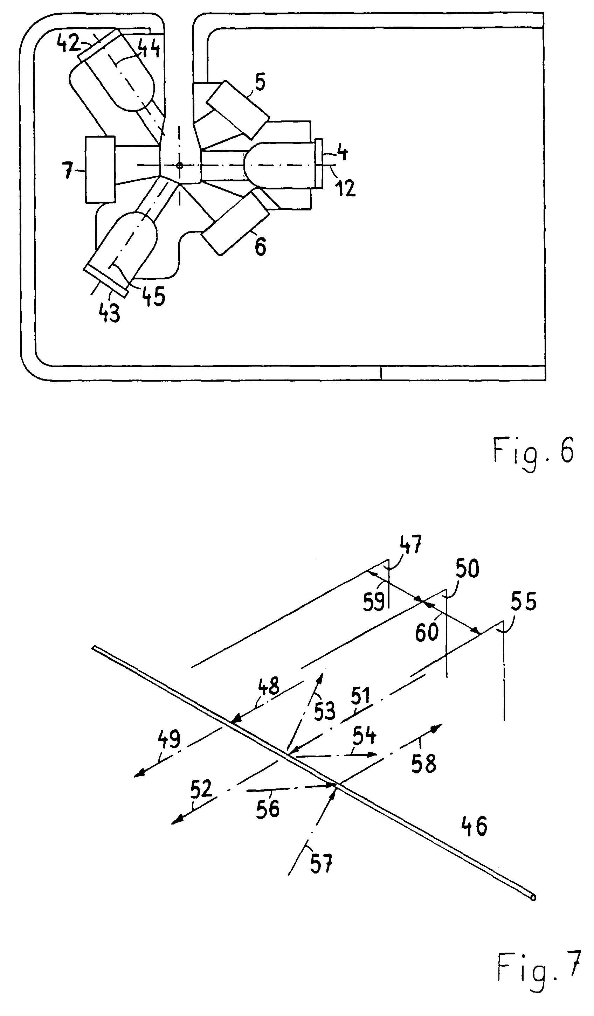

Fig. 5 zeigt zwei Signalverläufe 32, 33, die über einer horizontalen Achse 34, bzw, neben einer vertikalen Achse 35 aufgezeichnet sind. Auf der horizontalen Achse 34 sind Werte für einen Weg etwa entsprechend einem Abstand vom Eingang oder der seitlichen Begrenzungsfläche 40 (Fig. 1) eines Messspaltes 3 aufgetragen. Auf der vertikalen Achse 35 sind Werte eines elektrischen Signales aufgetragen, wie es von den Empfängern abgegeben wird. Demnach gibt der Signalverlauf 32 hier an, welche Werte der Empfänger für übertragenes Licht misst, wenn das Garn nach und nach von oben (gemäss Fig. 1) tiefer in den Messspalt 3 hineingeschoben wird. Der Signalverlauf 33 gibt entsprechend die Werte an, die zwei Empfänger von reflektiertem Licht in der Anordnung gemäss Fig. 1 in diesem Falle messen. Die grössten Werte werden gemessen, wenn das Garn 2 etwa in der in Fig. 1 gezeigten Stellung ist. Dann hat der Signalverlauf 32 einen Bereich 32', in dem die Werte etwa konstant bleiben. Beim Signalverlauf 33 ergibt sich im Bereiche 33' ein möglicherweise etwas ungleichmässiger Verlauf, der, wie nachfolgend noch erläutert wird, durch ungleiche Verteilung des reflektierten Lichts auf zwei Empfänger bewirkt ist. Mit 41 ist ein Signalverlauf bezeichnet, wie er auftritt, wenn nur ein einziger Empfänger für reflektiertes Licht vorgesehen ist.

Fig. 6 zeigt eine Darstellung eines Garnsensors entsprechend Fig. 1, wobei der Garnsensor aber neben der Lichtquelle 4 und den bekannten Empfängern 5, 6 und 7 zwei weitere Lichtquellen 42 und 43 mit optischen Achsen 44 und 45 aufweist. Die optischen Achsen dieser gegenüber Fig. 1 zusätzlichen Lichtquellen 44 und 45 oder der zugeordneten das Licht übertragenden Elemente liegen vorzugsweise im wesentlichen in der gleichen Ebene wie die bereits bekannten optischen Achsen 12, 13, 14 und 15. Es ist aber auch denkbar, einen Teil der nun bekannten optischen Achsen in eine erste und einen Teil in eine zweite Ebene zu legen.

Fig. 7 zeigt eine schematische Anordnung von mehreren Lichtquellen und Empfängern an einem Garn 46. Dabei sind die Lichtquellen, die Empfänger und die zugehörigen Elemente zum Übertragen des Lichts hier nur durch ihre optischen Achsen vertreten. In der als Beispiel gezeigten Anordnung erkennen wir in einer ersten Ebene 47 die optische Achse 48 einer Lichtquelle und die optische Achse 49 eines Empfängers. In einer dazu beabstandeten zweiten Ebene 50 liegen optische Achsen 51 einer Lichtquelle und 52, 53 und 54 von Empfängern, wie dies etwa aus der Fig. 1 bereits bekannt ist. In einer weiteren dazu beabstandeten Ebene 55 liegen die optischen Achsen 56, 57 von zwei Lichtquellen und die optische Achse 58 eines Empfängers. Die Ebenen 47, 50 und 55 sind vorzugsweise annähernd parallel zueinander und haben Abstände 59 und 60, die der Ausbildung der Elemente und des Platzbedarfs der Elemente Rechnung tragen, die durch die optischen Achsen hier vertreten sind.

Die Wirkungsweise des erfindungsgemässen Garnsensors ist wie folgt:
Durch eine an sich bekannte und hier nicht gezeigte Führung, wird dafür gesorgt, dass das Garn 2 sich im gezeigten Bereiche im Messspalt 3 aufhält, wenn es in seiner Längsrichtung durch diesen in ebenfalls bekannter Weise hindurchgezogen wird. Im Messspalt 3 wird das Garn 2 vom Lichtstrahl der Lichtquelle 4 belichtet. Licht das nicht auf das Garn 2 auftrifft und von diesem auch nicht genügend abgelenkt wird, trifft auf den Empfänger 7 auf und wird von diesem zu einem elektrischen Signal umgewandelt. Licht das vom Garn reflektiert wird, trifft auf die Empfänger 5 oder 6, sofern es in einem genügend grossen Winkel 35, 36 reflektiert wird. Danach werden die Signale aus den Empfängern 5, 6, 7 in an sich bekannter und wie beispielsweise in der genannten WO 93/13407 dargestellten Weise verarbeitet.

Falls das Garn sich aus seiner in Fig. 1 gezeigten Lage verschiebt, so spielt das für den Empfänger 7 in einem gewissen Bereiche keine Rolle, wie der Signalverlauf 32 im Bereiche 34 zeigt. Der Empfänger 5 erhält mehr reflektiertes Licht, wenn das Garn 2 im Messpalt 3 nach oben verschoben ist. Dieser Umstand drückt sich im Signalverlauf 33 im Buckel 37 aus. Der Empfänger 6 erhält mehr Licht, wenn das Garn 2 im Messspalt 3 nach unten verschoben ist. Das ergibt einen Buckel 38 im Signalverlauf 33. Im idealen Fall treten die Buckel 37 und 38 aber kaum hervor. Das kann dadurch erreicht werden, dass die Breite der lichtübertragenden Elemente 8, 9, 10 und 11 im Verhältnis zum Garndurchmesser und zur Position des Garns 2 im Messspalt 3 richtig abgestimmt wird, was beispielsweise durch Versuchsanordnungen ermittelt werden kann. Der leichte Einbruch des Signalverlaufes 33 im Bereiche 34 ist eben darauf zurückzuführen, dass ein Teil des vom Garn 2 reflektierten Lichtes nicht erfasst wird. Dieser Teil betrifft Licht, das in einem Winkel zurückgeworfen wird, der kleiner als die Winkel 35 und 36 ist.

Der erfindungsgemässe Gamsensor kann sowohl mit einem wie auch mit zwei oder mehr Empfängern für reflektiertes Licht versehen werden, wobei aber die Ausführung mit zwei zur optischen Achse 12 symmetrisch angeordneten Empfängern 5, 6 oder Elementen 10, 11 zum Übertragen des Lichts besonders vorteilhaft ist und einen gleichmässigeren Signalverlauf 33 ergibt.

Bei der Ausführung gemäss Fig. 6 hat man zusätzlich die Möglichkeit die Lichtquellen 4, 42, 43 so auszubilden, dass jede Lichtquelle Licht einer anderen Wellenlänge aussendet. So kann erkannt werden, ob Fremdmaterial wie Fremdfasern oder Verunreinigungen im Garn vorhanden sind. Durch geeignete Wahl des ausgesendeten Lichts kann gezielt nach bestimmten Fremdmaterialien gesucht werden.

Senden alle Lichtquellen gleiches Licht aus, so ist es möglich jede Lichtquelle nur für eine begrenzte Zeit leuchten zu lassen und sequentiell ein- und auszuschalten. So belichtet man das Garn jeweils aus einer anderen Richtung, so dass festgestellt werden kann, ob das Garn rund ist oder ob es flache Stellen aufweist.

Bei einer Ausführung gemäss Fig. 7 ist es beispielsweise denkbar, in der Ebene 47 eine erste Eigenschaft des Garns, beispielsweise seinen Durchmesser zu erfassen. In der Ebene 50 kann eine zweite Eigenschaft, beispielsweise der Gehalt an Fremdstoffen erfasst werden. In der weiteren Ebene 55 könnte beispielsweise die Beschaffenheit der Oberfläche des Garns 46 erfasst und eine Aussage darüber erzeugt werden, usw.

Der erfindungsgemässe Gamsensor kann natürlich auch für andere langgestreckte fadenartige Gebilde ausgebildet und verwendet werden. Der Begriff Gamsensor bezieht sich hier lediglich auf die häufigste Anwendung, die darin besteht in einem solchen Gebilde oder Garn Fremdstoffe oder Fremdkörper zu erkennen oder andere Eigenschaften wie Durchmesser, Gleichmässigkeit, Aufbau, Haarigkeit usw. anzugeben.

## Patentansprüche

1. Gamsensor (1) zum Abtasten eines, in seiner Längsrichtung in einem Messspalt (3), bewegten Garns (2) mit einem Lichtstrahl aus einer Lichtquelle (4), mit einem ersten Empfänger (7) für direkt übertragenes Licht, Elementen (8, 9) zum Übertragen des Lichts zwischen dem Messspalt, der Lichtquelle und dem Empfänger, wobei die optischen Achsen (12, 13) der Elemente zum Übertragen des Lichts zusammmen in einer Ebene liegen, die quer zum Gam steht, dadurch gekennzeichnet, dass mindestens zwei weitere Empfänger (5, 6) für Licht vorgesehen sind, das vom Gam reflektiert wird und dass die optischen Achsen (14, 15) der weiteren Empfänger ebenfalls quer zum Garn stehen und sich im Bereiche des Garns schneiden.

2. Gamsensor nach Anspruch 1, dadurch gekennzeichnet, dass die Elemente zum Übertragen des Lichts ein Fenster (16, 17, 18, 19) gegen den Messspalt aufweisen und dass die Fenster der optischen Elemente zum Übertragen des reflektierten Lichts zum Empfänger, senkrecht zur Bewegungsrichtung des Garns gesehen, neben der optischen Achse (12) der Lichtquelle liegen.

3. Gamsensor nach Anspruch 1, dadurch gekennzeichnet, dass zwischen den Empfängern, dem Messspalt und der Lichtquelle ein einstückig ausgebildetes Teil (25) zum Übertragen des Lichts vorgesehen ist, das einen Lichtschacht (26) für zugeführtes Licht und Lichtschächte (27, 28, 29) für weggeführtes Licht bildet.

4. Gamsensor nach Anspruch 3, dadurch gekennzeichnet, dass ein lichtdurchlässiger Körper (31) vorgesehen ist, der in den Teil (25) eingesetzt werden kann und als Einsatz in die Lichtschächte gedacht ist.

5. Gamsensor nach Anspruch 1, dadurch gekennzeichnet, dass weitere optische Achsen von weiteren Lichtquellen und Empfängern in weiteren Ebenen (47, 50, 55) liegen, die zueinander beabstandet sind.

6. Gamsensor nach Anspruch 1, dadurch gekennzeichnet, dass weitere Lichtquellen (42, 43) vorgesehen sind, deren optische Achsen (44, 45) in der Ebene liegen.

7. Gamsensor nach Anspruch 5 oder 6, dadurch gekennzeichnet, dass die Lichtquellen sequentiell angesteuert sind.

8. Garnsensor nach Anspruch 1, dadurch gekennzeichnet, dass die Empfänger zur Detektion von Fremdstoffen im Garn vorgesehen sind.

## Claims

1. Yarn sensor (1) for scanning a yarn (2), which is moving in its longitudinal direction in a measuring gap (3), with a light beam from a light source (4), having a first receiver (7) for directly transmitted light, elements (8, 9) for transmitting the light between the measuring gap, the light source and the receiver, wherein the optical axes (12, 13) of the elements for transmitting light lie together in one plane, which is situated at right angles to the yarn, characterized in that at least two further receivers (5, 6) are provided for light which is reflected by the yarn and that the optical axes (14, 15) of the further receivers are likewise situated at right angles to the yarn and intersect in the region of the yarn.

2. Yarn sensor according to claim 1, characterized in that the elements for transmitting the light comprise a window (16, 17, 18, 19) directed towards the measuring gap and that the windows of the optical elements for transmitting the reflected light to the receiver are situated - viewed at right angles to the direction of motion of the yarn - next to the optical axis (12) of the light source.

3. Yarn sensor according to claim 1, characterized in that an integrally constructed part (25) for transmitting the light is provided between the receivers, the measuring gap and the light source and forms a light shaft (26) for supplied light and light shafts (27, 28, 29) for light which is conveyed away.

4. Yarn sensor according to claim 3, characterized in that a transparent body (31) is provided, which may be inserted into the part (25) and is intended as an insert into the light shafts.

5. Yarn sensor according to claim 1, characterized in that further optical axes of further light sources and receivers are situated in further planes (47, 50, 55) which are spaced apart from one another.

6. Yarn sensor according to claim 1, characterized in that further light sources (42, 43) are provided, the optical axes (44, 45) of which are situated in the plane.

7. Yarn sensor according to claim 5 or 6, characterized in that the light sources are activated sequentially.

8. Yarn sensor according to claim 1, characterized in that the receivers are provided for detecting impurities in the yarn.

## Revendications

1. Capteur de fil (1) pour le balayage d'un fil (2) déplacé dans sa direction longitudinale dans une fente de mesure (3), avec un rayon lumineux provenant d'une source de lumière (4), avec un premier récepteur (7) pour la lumière transmise directement, des éléments (8, 9) pour la transmission de la lumière entre la fente de mesure, la source de lumière et le récepteur, où les axes optiques(12, 13) des éléments pour la transmission de la lumière se situent conjointement dans un plan qui est orienté transversalement au fil, caractérisé en ce que sont prévus au moins deux autres récepteurs (5, 6) pour la lumière qui est réfléchie par le fil et que les axes optiques (14, 15) des récepteurs supplémentaires s'étendent également transversalement au fil et se croisent au voisinage du fil.

2. Capteur de fil selon la revendication 1, caractérisé en ce que les éléments pour la transmission de la lumière présente une fenêtre (16, 17, 18, 19) contre la fente de mesure et que les fenêtres des éléments optiques pour la transmission de la lumière réfléchie vers le récepteur se situent, en regardant perpendiculairement à la direction de déplacement du fil, à côté de l'axe optique (12) de la source de lumière.

3. capteur de fil selon la revendication 1, caractérisé en ce qu'il est prévu entre les récepteurs, la fente de mesure et la source de lumière une partie (25) réalisée en une pièce pour la transmission de la lumière qui forme un puits de lumière (26) pour la lumière amenée et des puits de lumière (27, 28, 29) pour la lumière éloignée.

4. Capteur de fil selon la revendication 3, caractérisé en ce qu'il est prévu un corps (31) perméable à la lumière qui peut être placé dans la partie (25) et qui est prévu comme insert dans les puits de lumière.

5. Capteur de fil selon la revendication 1, caractérisé en ce que d'autres axes optiques d'autres sources de lumière et de récepteurs se situent dans d'autres plans (47, 50, 55) qui sont espacés les uns des autres.

6. Capteur de fil selon la revendication 1, caractérisé en ce que d'autres sources de lumière (42, 43) sont prévues, dont les axes optiques (44, 45) se situent dans le plan.

7. Capteur de fil selon la revendication 5 ou 6, caractérisé en ce que les sources de lumière sont commandées séquentiellement.

8. Capteur de fil selon la revendication 1, caractérisé en ce que les récepteurs sont prévus pour détecter des matières étrangères dans le fil.
